# EUROPÄISCHE PATENTANMELDUNG

(11) **EP 3 797 828 A1**
(43) Veröffentlichungstag der Anmeldung: **31.03.2021**
(21) Anmeldenummer: 19200202.0
(22) Anmeldetag: 27.09.2019
(51) Int. Cl.: A61N 1/375

(54) **LÖSBARE DICHTUNG FÜR MEDIZINISCHE IMPLANTATE**

(71) Anmelder: Dyconex AG, 8303 Bassersdorf (CH)
(72) Erfinder: Bihler, Eckardt, 8406 Winterthur (CH); Hauer, Marc, 8610 Uster (CH)
(74) Vertreter: Galander, Marcus

(57) **Zusammenfassung**

Die Erfindung betrifft ein medizinisches Implantat, mit: einer ersten Komponente aufweisend eine Oberfläche und eine Mehrzahl an elektrischen Kontakten, eine zweite Komponente aufweisend eine Oberfläche sowie eine Mehrzahl an elektrischen Kontakten, wobei jeder Kontakt der ersten Komponente einen zugeordneten Kontakt der zweiten Komponente elektrisch leitend kontaktiert, und einer Dichtung, die zum Abdichten der Kontakte zwischen den beiden Oberflächen angeordnet ist. Erfindungsgemäß ist vorgesehen, dass die Dichtung und die beiden Oberflächen aus einem thermoplastischen Material gebildet sind, wobei die Dichtung mit den beiden Oberflächen zum Abdichten der Kontakte verschmolzen ist und zum voneinander Trennen der beiden Komponenten aufschmelzbar ist. Weiterhin betrifft die Erfindung ein Verfahren zur Herstellung eines medizinischen Implantats.

## Beschreibung

Die vorliegende Erfindung betrifft ein medizinisches Implantat sowie ein Verfahren zur Herstellung eines solchen Implantats.

Dichtungen in medizinischen Implantaten müssen zuverlässig abdichten. Bei herkömmlichen Dichtungen aus einem gummiartigen Material wird die Abdichtung durch eine Deformation infolge einer einwirkenden Kraft erreicht. Um diese Kraft auszuüben, sind gewisse mechanische Anforderungen zu erfüllen. Diese machen das Verbindungssystem größer und kostspieliger.

Wenn ein bereits implantiertes elektronisches Implantat wieder entfernt werden soll, muss es möglich sein, die Elektrode vom Implantat zu trennen. Dazu werden Stecker verwendet. Innerhalb dieser Stecker muss der Kontaktbereich der Elektrodenleitungen vom Gewebe des Patienten abgedichtet werden.

Die derzeit auf dem Markt befindlichen Systeme verwenden oftmals Dichtungen, die über einen mechanischen Druck deformiert werden und auf diese Weise eine abdichtende Wirkung entfalten. Das erhöht die Komplexität des Systems und verhindert eine sichere Abdichtung über lange Zeiträume, da eine Diffusion entlang der Grenzflächen nicht ausgeschlossen werden kann. Aufgrund dieser Diffusion müssen die Kontakte im Stecker jeweils einzeln abgedichtet werden.

Der vorliegenden Erfindung liegt hiervon ausgehend die Aufgabe zugrunde, ein medizinisches Implantat sowie ein Verfahren zur Herstellung eines medizinischen Implantats bereitzustellen, die hinsichtlich der oben genannten Problematik verbessert sind.

Diese Aufgabe wird durch ein medizinisches Implantat mit den Merkmalen des Anspruch 1 sowie durch ein Verfahren mit den Merkmalen des Anspruchs 7 gelöst. Vorteilhafte Ausgestaltungen dieser Erfindungsaspekte sind in den entsprechenden Unteransprüchen angegeben und werden nachfolgend beschrieben.

Gemäß Anspruch 1 wird ein medizinisches Implantat offenbart, mit:
- einer ersten Komponente aufweisend eine Oberfläche und eine Mehrzahl an elektrischen Kontakten, wobei insbesondere auf der Oberfläche die Mehrzahl an elektrischen Kontakten angeordnet ist,
- einer zweiten Komponente aufweisend eine Oberfläche sowie eine Mehrzahl an elektrischen Kontakten, wobei jeder Kontakt der ersten Komponente einen zugeordneten Kontakt der zweiten Komponente elektrisch leitend kontaktiert, und
- einer Dichtung, die zum Abdichten der Kontakte zwischen den beiden Oberflächen angeordnet ist.

Erfindungsgemäß ist vorgesehen, dass die Dichtung und die beiden Oberflächen aus einem thermoplastischen Material gebildet sind, wobei die Dichtung mit den beiden Oberflächen zum Abdichten der Kontakte verschmolzen ist und zum voneinander Trennen der beiden Komponenten aufschmelzbar ist.

Mit anderen Worten sieht die Erfindung also eine thermische aktivierbare Dichtung vor, die einen vielpoligen Kontaktbereich abdichtet. Die Dichtung selbst und die Grenzflächen sind dabei vorzugsweise aus einem thermoplastischen Material gefertigt.

Durch das Einbringen der Temperatur und das Applizieren eines Druckes schmilzt die Dichtung auf, richtet sich mechanisch aus und verbindet sich mit den Grenzflächen bzw. Oberflächen der Komponenten. Nach dem Abkühlen der Dichtung kann der Druck wieder entfernt werden und die Verbindung bleibt bestehen.

Die Verbindung schützt den vielpoligen Kontaktbereich, d.h., die Kontakte der beiden Komponenten, nun vor den äußeren Einflüssen. Wenn die Kontakte bzw. die beiden Komponenten wieder getrennt werden müssen, wird die Dichtung wieder erhitzt und die beiden Komponenten werden durch eine Zug- oder Druckkraft wieder voneinander getrennt. Die Kontakte der beiden Komponenten weisen vorzugsweise jeweils eine Goldmetallisierung auf. Vorzugsweise ist gemäß einer Ausführungsform weiterhin vorgesehen, dass jeder Kontakt der ersten Komponente den zugeordneten Kontakt der zweiten Komponente mechanisch berührt und dadurch elektrisch leitend kontaktiert.

Die besagte aufschmelzbare Dichtung ist vorzugsweise ringförmig oder umlaufend ausgebildet und umgibt bzw. umläuft dabei die besagten Kontakte.

Weiterhin ist gemäß einer Ausführungsform der Erfindung vorgesehen, dass das thermoplastische Material ein Flüssigkristall-Polymer umfasst oder im Wesentlichen daraus besteht.

Weiterhin ist gemäß einer bevorzugten Ausführungsform der vorliegenden Erfindung vorgesehen, dass das medizinische Implantat zum Trennen der beiden Komponenten zumindest ein Heizelement aufweist, das dazu ausgebildet ist, das thermoplastische Material der Dichtung aufzuschmelzen. Gemäß einer Ausführungsform ist vorgesehen, dass das Heizelement als ein Heizleiter ausgebildet ist, welches dazu ausgebildet ist, das thermoplastische Material der Dichtung aufzuschmelzen, wenn ein vordefinierter elektrischer Strom durch den Heizleiter strömt. Hierzu weist der Heizleiter zum Beispiel zwei elektrische Kontakte auf, an die eine Spannungsquelle anschließbar ist. Gemäß einer alternativen Ausführungsform ist vorgesehen, dass das Heizelement dazu ausgebildet ist, das thermoplastische Material der Dichtung aufzuschmelzen, wenn das Heizelement einem Magnetfeld, insbesondere einem magnetischen Wechselfeld, oder hochfrequenten Radiowellen ausgesetzt wird.

Weiterhin ist gemäß einer Ausführungsform der Erfindung vorgesehen, dass das mindestens eine Heizelement, insbesondere der Heizleiter, in die Dichtung eingebettet ist oder auf einer der Oberflächen der Komponenten angeordnet ist.

Gemäß einer Ausführungsform ist weiterhin vorgesehen, dass das mindestens eine Heizelement, insbesondere der Heizleiter, auf der ersten Komponente oder auf der zweiten Komponente angeordnet ist.

Weiterhin ist gemäß einer Ausführungsform vorgesehen, dass das Implantat ein weiteres Heizelement, insbesondere einen Heizleiter, aufweist, wobei insbesondere das mindestens eine Heizelement auf der ersten Komponente angeordnet ist, und wobei das weitere Heizelement auf der zweiten Komponente oder in der Dichtung angeordnet ist.

Das weitere Heizelement ist dabei ebenfalls vorzugsweise in die Dichtung eingebettet oder auf der Oberfläche der zweiten Komponente angeordnet.

Die beiden Komponenten können dann voneinander getrennt werden, indem entweder das eine Heizelement oder das weitere Heizelement oder beide Heizelemente verwendet werden, um die Dichtung und/oder einen angrenzenden Bereich der jeweiligen Oberfläche aufzuschmelzen (beispielsweise Joulsche Wärme aufgrund des Stromes in einem betreffenden Heizleiter).

Weiterhin ist gemäß einer Ausführungsform der Erfindung vorgesehen, dass die Dichtung ein elastisch deformierbares Dichtungselement aufweist, das gegen die beiden Komponenten oder Oberflächen vorgespannt ist.

Gemäß einer Ausführungsform der Erfindung ist diesbezüglich vorzugsweise vorgesehen, dass die Dichtung einen inneren und einen äußeren Materialbereich aufweist, wobei die beiden Materialbereiche jeweils aus dem thermoplastischen Material geformt sind, und wobei das Dichtungselement zwischen den beiden Materialbereichen angeordnet ist.

Aufgrund der Vorspannung des elastisch deformierbaren bzw. deformierten Dichtungselements, drückt dieses Dichtungselement die beiden Komponenten auseinander, wenn die Dichtung bzw. die beiden Materialbereiche aufgeschmolzen werden (z.B. mittels eines der Heizelemente oder mittels einer extern erzeugten Wärme).

Gemäß einer weiteren Ausführungsform der Erfindung ist vorgesehen, dass in die Dichtung zumindest ein vorgespanntes Federelement eingebettet ist, so dass die beiden Komponenten mittels des mindestens einen Federelementes voneinander weggedrückt werden, wenn die Dichtung aufgeschmolzen wird.

Weiterhin ist gemäß einer bevorzugten Ausführungsform der Erfindung vorgesehen, dass die erste Komponente durch ein erstes Flachkabel gebildet ist. Gemäß einer weiteren Ausführungsform ist vorgesehen, dass die zweite Komponente durch ein zweites Flachkabel gebildet ist. Ein solches Flachkabel ist insbesondere dadurch gekennzeichnet, dass ein Leitungskörper des Flachkabels eine Breite aufweist, die größer ist als eine Höhe des Flachkabels senkrecht zu der Breite.

Insbesondere kann die Mehrzahl der Kontakte der zweiten Komponente gemäß einer Ausführungsform der Erfindung auf der besagten Oberfläche der zweiten Komponente angeordnet sein (z.B. wenn die beiden Komponenten durch Flachkabel gebildet sind).

Gemäß einer alternativen bevorzugten Ausführungsform ist vorgesehen, dass die erste Komponente durch ein Flachkabel gebildet ist und dass die zweite Komponente durch ein implantierbares medizinisches Gerät gebildet ist, wobei die Kontakte der zweiten Komponente Kontakte einer Durchführung des medizinischen Geräts sind. Über die Durchführung des medizinischen Gerätes ist eine Mehrzahl an elektrischen Leitern des Geräts aus einem Gehäuse des Geräts herausgeführt. Bei dem medizinischen Gerät kann es sich zum Beispiel um einen Pulsgenerator zur Neurostimulation, Herzschrittmacher oder Kardioverter-Defibrillator handeln.

Die elektrischen Leiter des Geräts sind insbesondere in einen Isolator (z.B. einen keramischen Isolator) der Durchführung eingebettet, so dass insbesondere die besagten Kontakte der zweiten Komponente freiliegen und/oder aus dem Isolator herausstehen.

Im Falle einer Durchführung ist gemäß einer Ausführungsform der Erfindung vorgesehen, dass sich die besagte Oberfläche der zweiten Komponente um die Kontakte der zweiten Komponente herum erstreckt. Die zwischen den beiden Oberflächen der Komponenten auszubildende Dichtung kann somit wiederum die einander kontaktierenden Kontakte der Komponenten abdichten.

Ein weiterer Aspekt der vorliegenden Erfindung betrifft ein Verfahren zur Herstellung eines medizinischen Implantats, aufweisend die Schritte:
- Bereitstellen einer ersten und einer zweiten Komponente des medizinischen Implantats, wobei die erste Komponente eine Oberfläche und eine Mehrzahl an elektrischen Kontakten aufweist, wobei insbesondere auf der Oberfläche die Mehrzahl an elektrischen Kontakten angeordnet ist, und wobei die zweite Komponente eine Oberfläche sowie eine Mehrzahl an elektrischen Kontakten aufweist, und wobei die Oberflächen aus einem thermoplastischen Material gebildet sind, und
- Gegeneinander Drücken der beiden Komponenten und Aufschmelzen einer aus dem thermoplastischen Material bestehenden und zwischen den beiden Oberflächen angeordneten Dichtung, so dass sich die Dichtung mit den beiden Oberflächen verbindet, und so dass jeder Kontakt der ersten Komponente einen zugeordneten Kontakt der zweiten Komponente elektrisch leitend kontaktiert.

Nach einem Aushärten der Dichtung sind die beiden Komponenten vorzugsweise fest miteinander verbunden.

Gemäß einer Ausführungsform des Verfahrens ist vorgesehen, dass die Dichtung (zumindest vor dem besagten Aufschmelzen) zwei aus dem thermoplastischen Material gebildete Materialbereiche aufweist.

Weiterhin ist gemäß einer Ausführungsform des Verfahrens vorgesehen, dass die Materialbereiche vor dem Aufschmelzen an der Oberfläche der ersten Komponente oder an der Oberfläche der zweiten Komponente festgelegt sind.

Weiterhin ist gemäß einer Ausführungsform des Verfahrens vorgesehen, dass zwischen den beiden Materialbereichen ein elastisch deformierbares Dichtungselement angeordnet wird bzw. ist, wobei das Dichtungselement beim gegeneinander Drücken (sowie beim Aufschmelzen der Dichtung) der beiden Komponenten zur Anlage an die beiden Oberflächen gelangen kann und gegen die beiden Oberflächen vorgespannt werden kann. Das besagte Dichtungselement kann z.B. aus einem Silikon bestehen (siehe auch oben). Weiterhin ist gemäß einer Ausführungsform des Verfahrens vorgesehen, dass das Dichtungselement ringförmig bzw. umlaufend ausgebildet ist und die Kontakte lateral umgibt bzw. umläuft.

Gemäß einer weiteren Ausführungsform des Verfahrens vorgesehen, dass der eine Materialbereich vor dem Aufschmelzen an der Oberfläche der ersten Komponente festgelegt ist, wobei der andere Materialbereich vor dem Aufschmelzen an der Oberfläche der zweiten Komponente festgelegt ist.

Weiterhin ist gemäß einer Ausführungsform des Verfahrens vorgesehen, dass beim Aufschmelzen der Dichtung zumindest ein Federelement in die Dichtung eingebettet wird und beim gegeneinander Drücken der beiden Komponenten gegen diese vorgespannt wird.

Das Dichtungselement bzw. die beiden Materialbereiche können für den Fall, dass keine der beiden Komponenten einen Heizleiter aufweist, mittels extern erzeugter Hitze aufgeschmolzen werden.

Weiterhin ist gemäß einer Ausführungsform des Verfahrens vorgesehen, dass die Dichtung (insbesondere die beiden Materialbereiche) mit Hilfe eines Heizelements aufgeschmolzen wird, der auf der ersten Komponente angeordnet ist und/oder dass die Dichtung (insbesondere die beiden Materialbereiche) mit Hilfe eines Heizelements aufgeschmolzen wird, der auf der zweiten Komponente angeordnet ist. Das Heizelement kann dabei eine interne Energiequelle (Batterie für einen Heizleiter) aufweisen oder über durch eine externe Energiequelle gespeist werden. Die externe Energiequelle kann dabei das Heizelement, insbesondere einen Heizleiter, mit elektrischer Energie versorgen. Alternativ kann aber auch Energie über ein Magnetfeldes, insbesondere ein magnetisches Wechselfeld, oder über eine RF-Koppelung (hochfrequente Radiowellen) eingebracht werden.

Gemäß einer Ausführungsform des Verfahrens kann das Heizelement der ersten Komponente in die Dichtung eingebettet oder auf der Oberfläche der ersten Komponente angeordnet sein.

Weiterhin ist gemäß einer Ausführungsform des Verfahrens vorgesehen, dass das Heizelement der zweiten Komponente in die Dichtung eingebettet ist oder auf der Oberfläche der zweiten Komponente angeordnet ist.

Weiterhin ist gemäß einer Ausführungsform des Verfahrens vorgesehen, dass die beiden Komponenten mittels eines Werkzeugs gegeneinandergedrückt werden, das zum Ausüben einer Kraft auf die beiden Komponenten einen schraubbaren Kolben aufweist.

Weiterhin kann das Werkzeug ein Federelement aufweisen, insbesondere in Form eines Blattfederrings oder eine Membranfeder, wobei das Federelement zwischen dem Kolben und der ersten oder der zweiten Komponente angeordnet wird, so dass die mittels des Kolbens erzeugbare Kraft über das Federelement des Werkzeugs in die erste bzw. die zweite Komponente einleitbar ist.

Weiterhin ist gemäß einer Ausführungsform des Verfahrens vorgesehen, dass die erste Komponente durch ein erstes Flachkabel gebildet ist. Weiterhin ist gemäß einer Ausführungsform des Verfahrens vorgesehen, dass auch die zweite Komponente durch ein (zweites) Flachkabel gebildet ist.

Weiterhin ist gemäß einer Ausführungsform des Verfahrens vorgesehen (siehe oben), dass die Mehrzahl der Kontakte der zweiten Komponente auf der besagten Oberfläche der zweiten Komponente angeordnet sind (z.B. wenn die beiden Komponenten durch Flachkabel gebildet sind).

Gemäß einer alternativen Ausführungsform des Verfahrens ist vorgesehen, dass die erste Komponente durch ein Flachkabel gebildet ist, und dass die zweite Komponente durch ein implantierbares medizinisches Gerät gebildet ist, wobei die Kontakte der zweiten Komponente Kontakte einer Durchführung des medizinischen Geräts sind (siehe auch oben).

Im Falle einer Durchführung ist gemäß einer Ausführungsform des Verfahrens vorgesehen, dass sich die besagte Oberfläche der zweiten Komponente um die Kontakte der zweiten Komponente herum erstreckt. Die zwischen den beiden Oberflächen der Komponenten auszubildende Dichtung kann somit wiederum die einander kontaktierenden Kontakte der Komponenten abdichten.

Bei dem medizinischen Gerät kann es sich zum Beispiel um einen Pulsgenerator zur Neurostimulation, einen Herzschrittmacher oder einen Kardioverter-Defibrillator handeln.

Weiterhin ist gemäß einer Variante des Verfahrens vorgesehen, dass die Dichtung zum Trennen der beiden Komponenten voneinander durch Einwirkung von Wärme wieder aufgeschmolzen wird, wobei die Wärme auf eine der folgenden Weisen erzeugt wird: mittels einer externen Wärmequelle, mittels des Heizelement der ersten Komponente und/oder mittels des Heizelements der zweiten Komponente.

Im Folgenden sollen Ausführungsformen sowie weitere Merkmale und Vorteile der Erfindung anhand der Figuren erläutert werden. Es zeigen:
- Fig. 1: eine schematische Darstellung einer Ausführungsform der Erfindung, wobei die Dichtung und der Heizleiter anfänglich auf derselben Oberfläche einer Komponente des Implantats festgelegt sind;
- Fig. 2: eine schematische Darstellung einer Ausführungsform der Erfindung, wobei die Dichtung und der Heizleiter anfänglich auf Oberflächen unterschiedlicher Komponenten des Implantats festgelegt sind;
- Fig. 3: eine schematische Darstellung einer Ausführungsform der Erfindung, wobei der Heizleiter in die Dichtung integriert bzw. eingebettet ist;
- Fig. 4: das Verschmelzen der Dichtung gemäß Figur 3 mit den Oberflächen der beiden Komponenten des Implantats;
- Fig. 5: zeigt eine Draufsicht auf eine erste Komponente (Flachkabel) einer Ausführungsform eines erfindungsgemäßen medizinischen Implantats mit einer thermoplastischen Dichtung, die ein zusätzliches elastisch deformierbares Dichtungselement (z.B. aus einem Silikon) aufweist;
- Fig. 6: eine Schnittansicht des in der Figur 5 gezeigten Flachkabels;
- Fig. 7: zeigt neben der ersten Komponente der Figuren 5 und 6 eine zweite Komponente in Form eines zweiten Flachkabels sowie ein Werkzeug zum gegeneinander Drücken der beiden Komponenten bzw. Flachkabel beim Aufschmelzen der Dichtung;
- Fig. 8: zeigt die beiden im Werkzeug angeordneten Flachkabel gemäß Figuren 5 bis 7 nach einem Verschmelzen der Dichtung mit den Oberflächen der Flachkabel zur Abdichtung der Kontakte der Flachkabel;
- Fig. 9: zeigt eine Draufsicht auf eine erste Komponente (Flachkabel) einer weiteren Ausführungsform eines erfindungsgemäßen medizinischen Implantats mit einer thermoplastischen Dichtung, die ein zusätzliches elastisch deformierbares Dichtungselement (z.B. aus einem Silikon) aufweist, wobei die erste Komponente weiterhin einen Heizleiter zum Aufschmelzen der Dichtung aufweist;
- Fig. 10: eine Schnittansicht des in der Figur 9 gezeigten Flachkabels;
- Fig. 11: zeigt neben der ersten Komponente der Figuren 9 und 10 eine zweite Komponente in Form eines zweiten Flachkabels mit einem Heizleiter sowie ein Werkzeug zum gegeneinander Drücken der beiden Komponenten bzw. Flachkabel beim Aufschmelzen der Dichtung;
- Fig. 12: zeigt die beiden im Werkzeug angeordneten Flachkabel gemäß Figuren 9 bis 11 nach einem Verschmelzen der Dichtung mit den Oberflächen der Flachkabel zur Abdichtung der Kontakte der Flachkabel;
- Fig. 13: zeigt eine weitere Ausführungsform eines erfindungsgemäßen medizinischen Implantats mit einer ersten und einer zweiten Komponente in Form eines Flachkabels, wobei an dem jeweiligen Flachkabel ein Materialbereich und ein Federelement der herzustellenden Dichtung festgelegt ist; weiterhin wird das Werkzeug zum gegeneinander Drücken der beiden Komponenten bzw. Flachkabel beim Aufschmelzen der Materialbereiche der Dichtung gezeigt;
- Fig. 14: zeigt die beiden im Werkzeug angeordneten Flachkabel gemäß Figur 13 nach einem Verschmelzen der Materialbereiche der Dichtung mit den Oberflächen der Flachkabel zur Abdichtung der Kontakte der Flachkabel, wobei die in die Dichtung eingebetteten Federelemente gegen die beiden Flachkabel vorgespannt sind;
- Fig. 15: zeigt das aus dem Werkzeug herausgenommene Implantat der Figur 14;
- Fig. 16: zeigt eine Draufsicht auf eine erste Komponente (Flachkabel) einer weiteren Ausführungsform eines erfindungsgemäßen medizinischen Implantats mit einer thermoplastischen Dichtung und einem Heizleiter zum Aufschmelzen der Dichtung;
- Fig. 17: zeigt eine Schnittansicht der ersten Komponente bzw. des ersten Flachkabels gemäß Figur 16, das mit einem zweiten Flachkabel des Implantats über die mit den Oberflächen der Flachkabel verschmolzene Dichtung verbunden ist;
- Fig. 18: zeigt eine weitere Ausführungsform eines erfindungsgemäßen medizinischen Implantats mit einer ersten Komponente in Form eines Flachkabels und einer zweiten Komponente in Form eines implantierbaren medizinischen Geräts, wobei die beiden Komponenten über eine mittels externer Wärme aufschmelzbare thermoplastische Dichtung miteinander verbunden sind und in einem Werkzeug zum gegeneinander Drücken der beiden Komponenten angeordnet sind, wobei das Werkzeug temporär an einer Außenseite des medizinischen Geräts festgelegt ist;
- Fig. 19: zeigt eine weitere Ausführungsform eines erfindungsgemäßen medizinischen Implantats mit einer ersten Komponente in Form eines Flachkabels und einer zweiten Komponente in Form eines implantierbaren medizinischen Geräts, wobei die beiden Komponenten über eine mittels eines Heizleiters aufschmelzbare thermoplastische Dichtung miteinander verbunden sind und in einem Werkzeug zum gegeneinander Drücken der beiden Komponenten angeordnet sind, wobei das Werkzeug temporär an einer Außenseite des medizinischen Geräts festgelegt ist;
- Fig. 20: zeigt das Implantat gemäß Figur 19 nach einem Entfernen des Werkzeugs;
- Fig. 21: zeigt eine weitere Ausführungsform eines erfindungsgemäßen medizinischen Implantats mit einer ersten Komponente in Form eines Flachkabels und einer zweiten Komponente in Form eines implantierbaren medizinischen Geräts, wobei an dem Gerät eine mittels Heizleiter aufschmelzbare Dichtung und ein Federelement vorgesehen sind; weiterhin wird das Werkzeug zum gegeneinander Drücken der beiden Komponenten beim Aufschmelzen der Dichtung gezeigt;
- Fig. 22: zeigt das Implantat gemäß Figur 21 mit ausgebildeter Dichtung, die mit den Oberflächen der beiden Komponenten verschmolzen ist, wobei das Werkzeug an der zweiten Komponente in Form des medizinischen Geräts festgelegt ist;
- Fig. 23: zeigt das Implantat gemäß Figur 22 nach einem Entfernen des Werkzeugs; und
- Fig. 24: zeigt eine Abwandlung des in der Figur 23 gezeigten Implantats nach einem Entfernen des Werkzeugs.

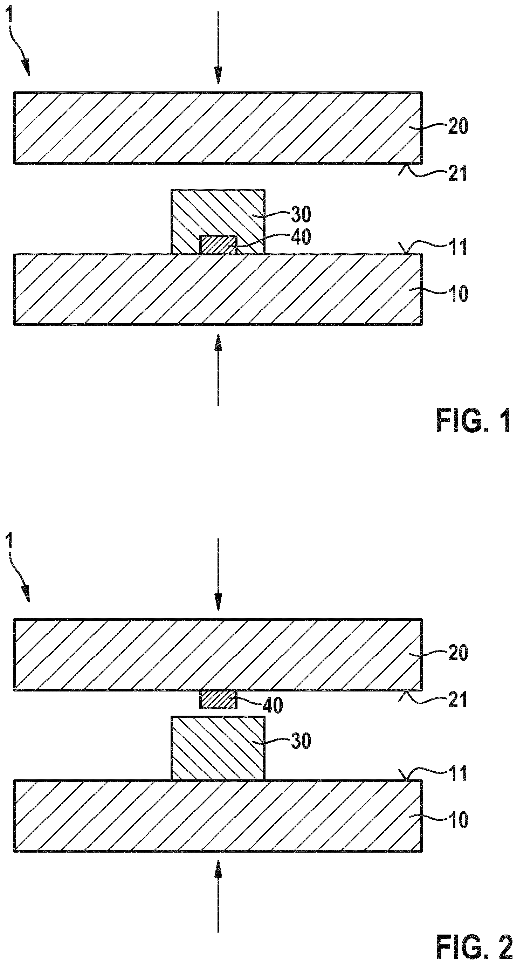

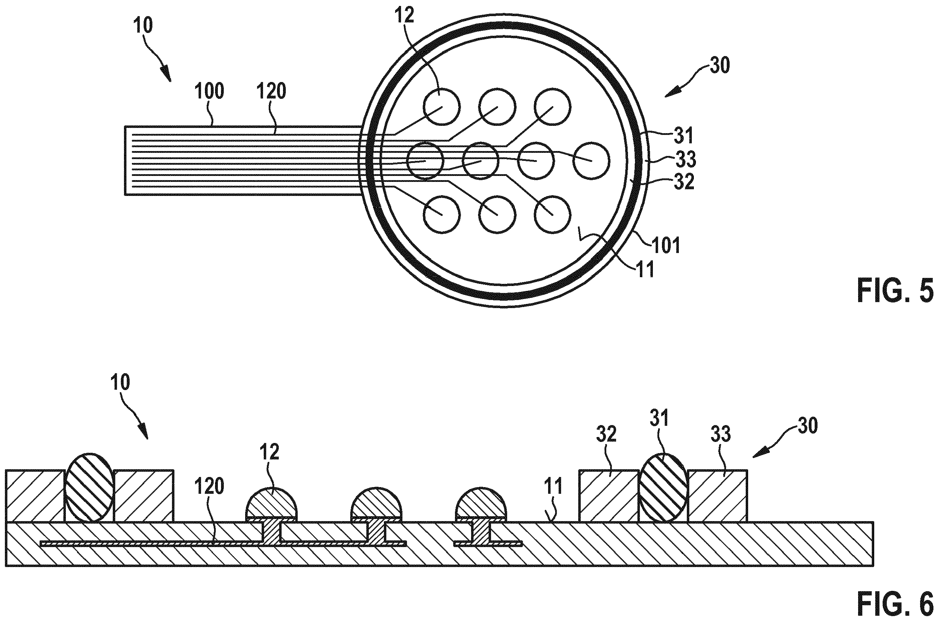

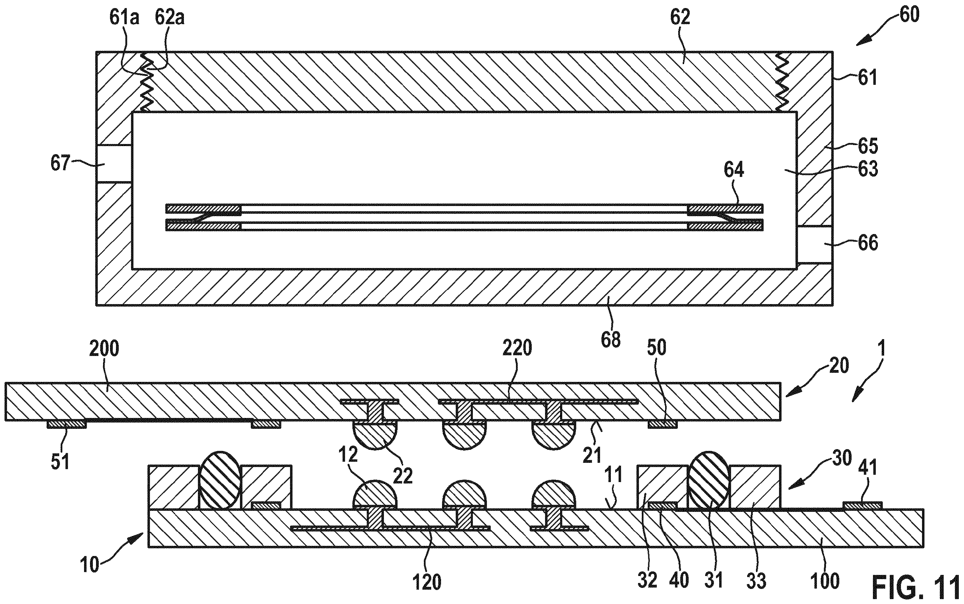

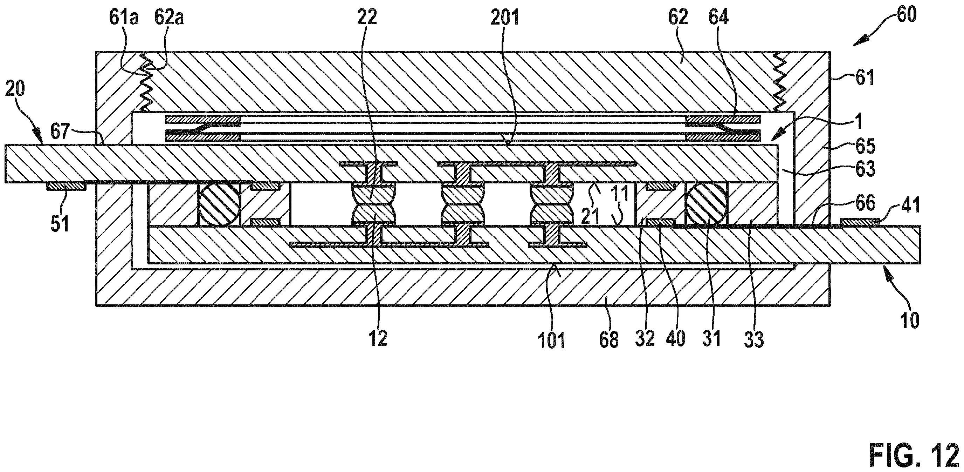

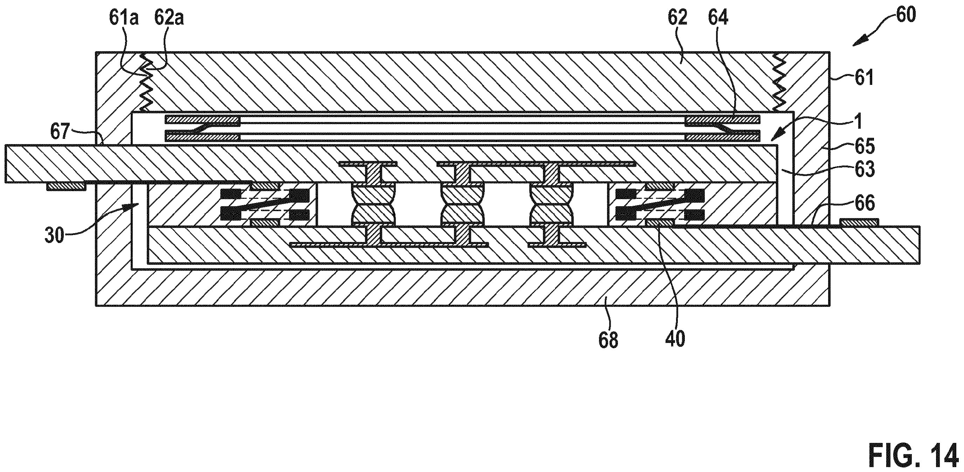

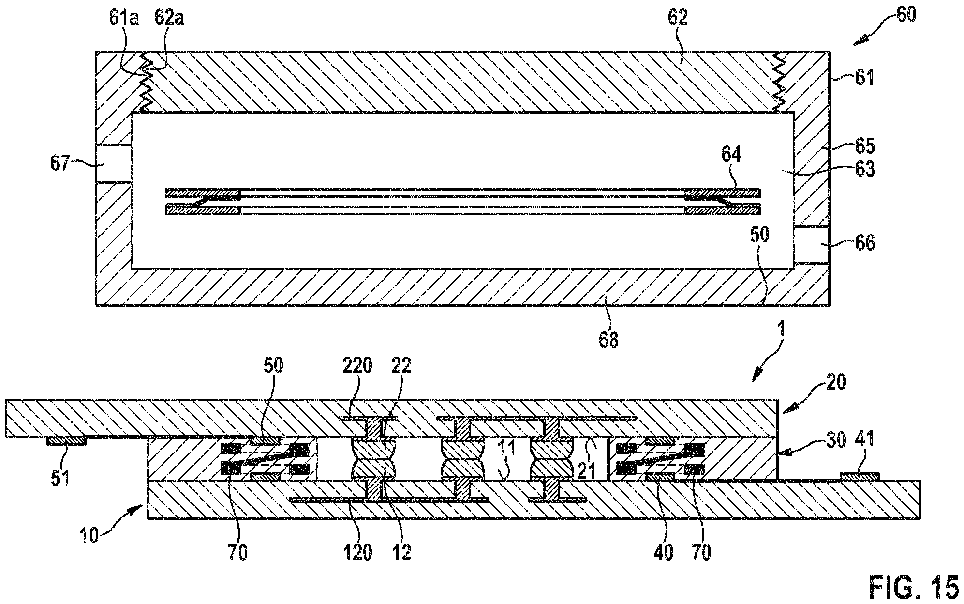

Die Figuren 1 bis 4 stellen schematisch das Prinzip der Herstellung einer Abdichtung zwischen zwei Komponenten 10, 20 eines medizinischen Implantats 1 dar. Dieses weist eine ersten Komponente 10 und eine zweite Komponente 20 auf, bei denen es sich z.B. um Flachkabel 10, 20 handeln kann. Eine der beiden Komponenten kann auch durch ein implantierbares medizinisches Gerät 20 gebildet sein, z.B. in Form eines Pulsgenerators (siehe unten). Die beiden Komponenten 10, 20 weisen jeweils eine Oberfläche 11, 21 auf, auf der jeweils Kontakte 12, 22 angeordnet sind (z.B. in Form von Kontaktarrays), wobei die Kontakte 12 der ersten Komponente 10 mit den Kontakten 22 der zweiten Komponente 20 elektrisch leitend verbunden sind, und zwar durch mechanischen Kontakt. Die beiderseitigen Kontakte 12, 22 weisen hierzu vorzugsweise eine Goldmetallisierung auf. Um nun eine zuverlässige, bei Implantaten 1 notwendige, Abdichtung dieser Kontakte 12, 22 sicherzustellen, weist das erfindungsgemäße Implantat 1 eine Dichtung 30 auf, die sich entlang der Oberflächen 11, 21 um die einander berührenden Kontakte 12, 22 herum erstreckt. Die Positionen der Kontakte 12, 22 sind der Übersichtlichkeit halber in den Figuren 1 bis 4 nicht gezeigt, können jedoch den in den Figuren 5 bis 24 beschriebenen Ausführungsformen entnommen werden.

Erfindungsgemäß ist vorgesehen, dass die Dichtung 30 und die beiden Oberflächen 11, 21 aus einem thermoplastischen Material gebildet sind (siehe z. B. oben), wobei die Dichtung 30 mit den beiden Oberflächen 11, 21 zum Abdichten der Kontakte 12, 22 verschmolzen ist und zum voneinander Trennen der beiden Komponenten 10, 20 aufschmelzbar ist. D.h., die über die Dichtung 30 fest miteinander verbundenen Komponenten können durch Aufschmelzen der Dichtung 30 bei Bedarf wieder voneinander getrennt werden.

Wie anhand der Fig. 1 schematisch ersichtlich ist, kann sich die Dichtung 30 bzw. ein entsprechender Materialbereich aus einem thermoplastischen Material bereits direkt auf einer der Grenz- bzw. Oberflächen befinden, hier die Oberfläche der ersten Komponente 10. Der Heizleiter 40 kann dabei auch auf der Oberfläche 11 angeordnet sein bzw. kann in die Dichtung 30 eingebettet sein. Hierdurch kann die Hitze des Heizleiters den Thermoplasten gut durchdringen.

Gemäß Fig. 2 kann alternativ der Heizleiter 40 auf der einen Grenz- bzw. Oberfläche 21 angeordnet sein, während die aufzuschmelzende Dichtung/Materialbereich 30 auf der gegenüberliegenden Grenz- bzw. Oberfläche 11 angeordnet ist.

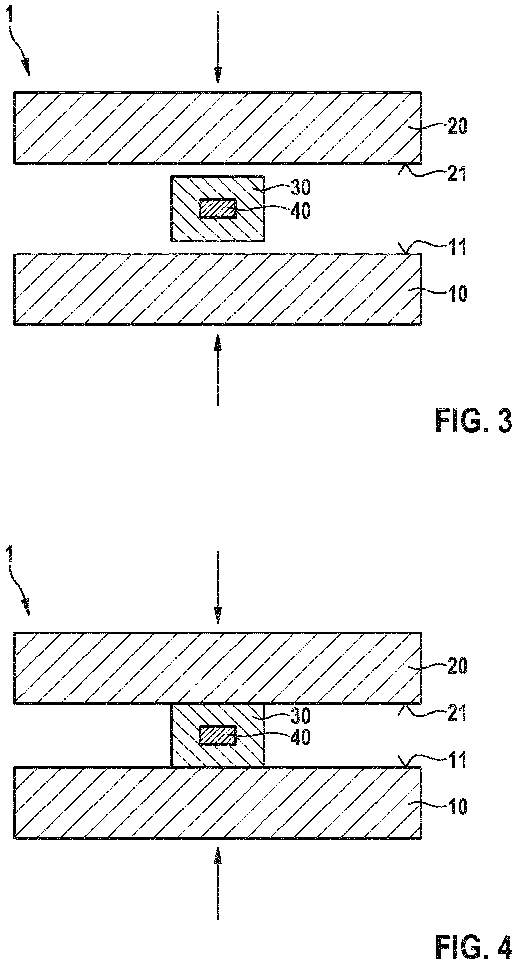

Gemäß Fig. 3 kann die Dichtung 30 anfangs auch ein separates Bauteil bilden, wobei hier der Heizleiter 40 in die Dichtung 30 eingebettet sein kann. In dieser Variante ist die Dichtung also gewissermaßen unabhängig von den Grenz- bzw. Oberflächen 11, 21.

Figur 4 zeigt in schematischer Art das Aufschmelzen der Dichtung 30 mittels des Heizleiters 40. Beim Aufschmelzen der thermoplastischen Dichtung 30 in Verbindung mit den thermoplastischen Grenz- bzw. Oberflächen 11, 21 verschwinden die Grenzflächen, d.h., die Dichtung 30 verbindet sich mit den Oberflächen 11, 21. Dadurch kann eine bessere Abdichtung erreicht werden. Hierdurch wird es möglich, mittels einer einzelnen Dichtung 30 ein ganzes Elektrodenarray 12, 22 vor der Umgebung abzudichten.

Wie in den Figuren 1 bis 4 gezeigt wird, kann die Dichtung 30 mittels eines Heizleiters 40 aufgeschmolzen werden. Es ist jedoch auch möglich, die Dichtung 30 mit extern erzeugter Wärme aufzuschmelzen. Eine entsprechende Ausführungsform der Erfindung ist in den Figuren 5 bis 8 dargestellt. Danach weist das medizinische Implantat 1 zwei Komponenten 10, 20 in Form von Flachkabeln 10, 20 auf, deren Kontakte 12, 22 miteinander elektrisch leitend verbunden werden sollen, wobei die Kontakte 12, 22 gleichzeitig mittels der thermisch aktivierbaren Dichtung 30 abgedichtet werden sollen.

Das erste Flachkabel 10 ist in den Figuren 5 und 6 gezeigt und weist einen Leitungskörper 100 auf, der die einzelnen elektrischen Leiter 120 des Flachkabels 10 umgibt. Das erste Flachkabel 10 weist weiterhin einen z.B. kreisförmigen Endabschnitt 101 auf, mit einer Oberfläche 11, auf der eine Vielzahl an Kontakten 12 angeordnet sind, die vorzugsweise eine Goldmetallisierung aufweisen. Jeder Kontakt 12 ist mit einem der Leiter 120 verbunden.

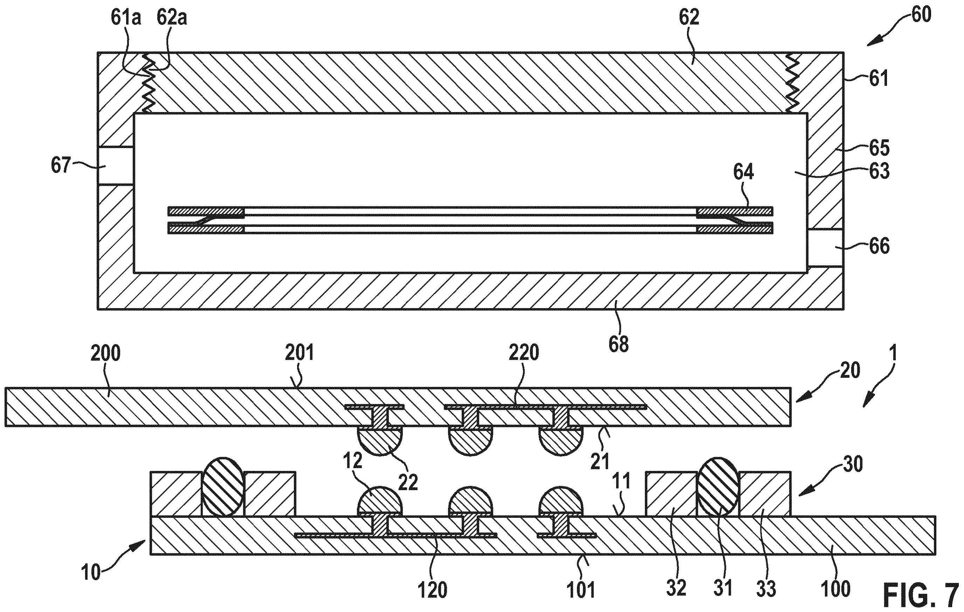

Gemäß Fig. 5 sind die Kontakte 12 vor dem Verbinden des ersten Flachkabels 10 mit dem zweiten Flachkabel 20 (vgl. Fig. 7) von einer Dichtung 30 umgeben. Diese thermoplastische Dichtung 32, 33 weist gemäß den Figuren 5 bis 7 zwei aus einem thermoplastischen Material bestehende Materialbereiche 32, 33 auf, nämlich einen umlaufenden bzw. ringförmigen inneren Materialbereich 32 und einen umlaufenden bzw. ringförmigen äußeren Materialbereich 33, wobei zwischen den beiden Materialbereichen 32, 33 eine Nut vorhanden ist, in der ein elastisch deformierbares Dichtungselement 31 (z.B. ein O-Ring aus einem Silikon) angeordnet ist. Die Materialbereiche 32, 33 stehen von der Oberfläche 11 des ersten Flachkabels 10 ab, die ebenfalls aus dem thermoplastischen Material gebildet ist.

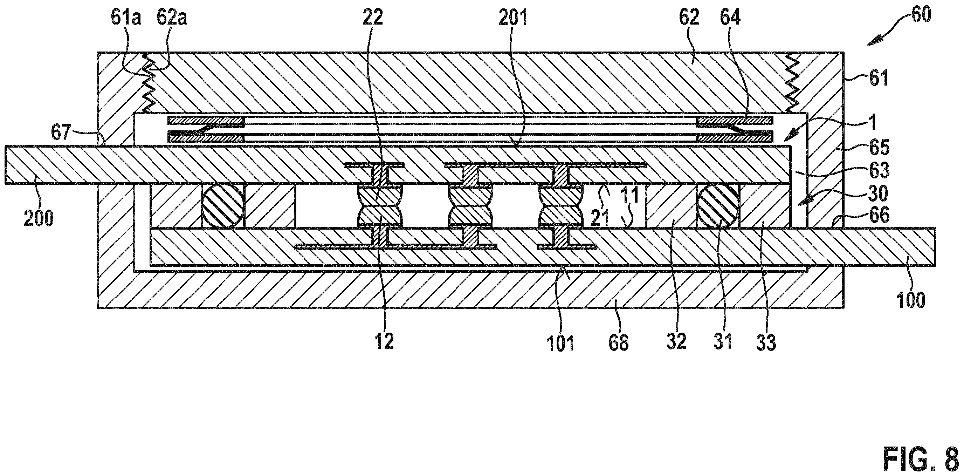

Die beiden Flachkabel 10, 20 werden nun zum Verbinden bzw. zum Herstellen des Implantats 1 gemäß Figur 7 aufeinander gelegt und mit einem Werkzeug 60 gegeneinander gedrückt, wobei weiterhin die Dichtung 30 aufgeschmolzen wird (z.B. durch Zufuhr externer Wärme) so dass sich die beiden Materialbereiche 32, 33 der Dichtung 30 mit den beiden thermoplastischen Oberflächen 11, 21 der Flachkabel 10, 20 verbinden. Hierbei wird gemäß Figur 8 das Dichtungselement 31 in die Dichtung 30 eingebettet und gegen die beiden Komponenten 10, 20 des Implantats 1 vorgespannt. Bei einem späteren Aufschmelzen der Dichtung 30 drückt dann das Dichtungselement 31 die beiden Komponenten zum besseren Trennen auseinander.

Das Werkzeug 60 weist zum gegeneinander Drücken der beiden Komponenten 10, 20 bzw. Flachkabel 10, 20 des Implantats 1 ein Gehäuse 61 mit einem Innengewinde 61a auf, in das ein Außengewinde 62a eines entsprechend schraubbaren Kolbens 62 eingreift. Das Gehäuse 61 weist eine seitliche Wandung 65 auf sowie einen dem Kolben 62 gegenüberliegenden Boden 68 auf, der mit der Wandung 65 verbunden ist. Weiterhin weist die Wandung 65 Ausschnitte 66, 67 auf, durch die Teile der Flachkabel 10, 20 (z.B. Abschnitte des jeweiligen Leitungskörpers 100, 200) aus einem Innenraum 63 des Werkzeugs 60 bzw. Gehäuses 61 herausführbar sind. Der Innenraum 63 nimmt somit vorzugsweise lediglich die aufeinander liegenden Endabschnitte 101, 201 der Flachkabel 10, 20 auf. Die beiden Flachkabel 10, 20 bzw. Endabschnitte 101, 201 sind dabei zwischen dem Kolben 62 und dem Boden 68 angeordnet. Weiterhin ist bevorzugt zwischen dem Kolben 62 und dem oberen zweiten Flachkabel 20 ein Federelement 64, insbesondere in Form eines Blattfederrings oder einer Membranfeder, angeordnet. Wird nun der Kolben 62 in das Gehäuse 61 reingeschraubt, drückt der Kolben 62 über das Federelement 64 gegen das zweite Flachkabel 20, das wiederum über die Dichtung 30 gegen das erste Flachkabel 10 drückt. Durch das Aufschmelzen der Dichtung 30 bzw. der thermoplastischen Materialbereiche 32, 33 verbinden sich diese mit den Oberflächen 11, 21 und die einander gegenüberliegenden Kontakte 12, 22 der Flachkabel 10, 20 kommen aufeinander zu liegen, so dass sie elektrisch leitend in Kontakt stehen.

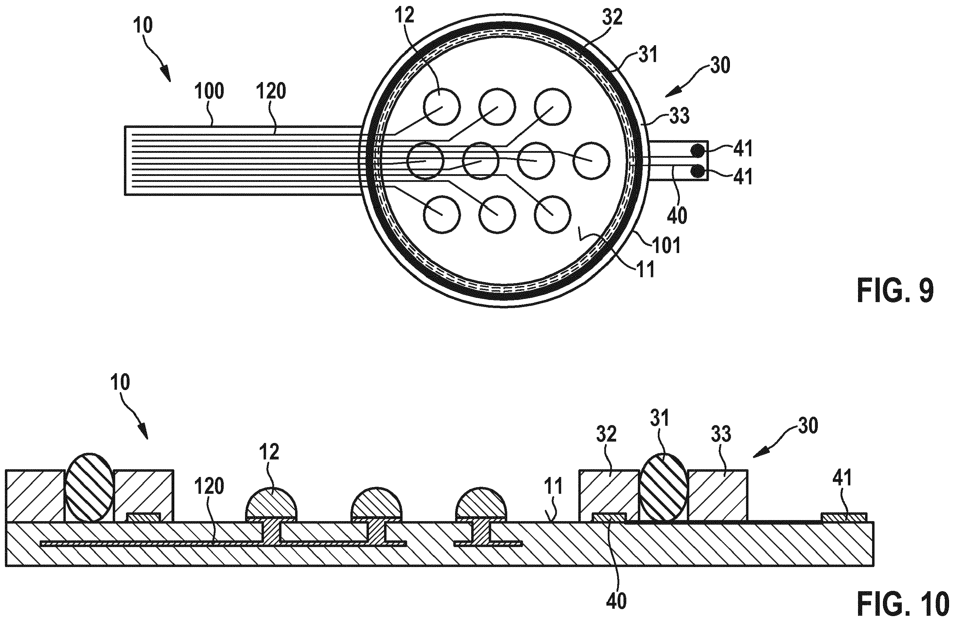

Die Figuren 9 bis 12 zeigen eine Abwandlung der in den Figuren 5 bis 8 gezeigten Ausführungsform, wobei hier im Unterschied zu den Figuren 5 bis 8 in das erste Flachkabel 10 sowie in das zweite Flachkabel 20 jeweils ein Heizleiter 40 bzw. 50 integriert ist. Der Heizleiter 40 des ersten Flachkabels 10 ist dabei unterhalb der aufzuschmelzenden Dichtung 30 angeordnet, wohingegen der Heizleiter 50 des zweiten Flachkabels 20 auf der Oberfläche 21 angeordnet ist und später - nach dem Verbinden der beiden Flachkabel 10, 20 - ebenfalls durch die Dichtung 30 verdeckt wird. Jeder der beiden Heizleiter 40, 50 kann daher nach einem Verschmelzen der Dichtung 30 mit den Oberflächen 11, 21 dazu verwendet werden, die ausgehärtete Dichtung 30 wieder aufzuschmelzen.

Damit die Heizleiter 40, 50 mit Spannung versorgt werden können, während die Endabschnitte 101, 201 der Flachkabel 10, 20 im Innenraum 63 des Werkzeugs 60 angeordnet sind, sind die Kontakte 41, 51 der Heizleiter 40, 50 z.B. durch die Ausschnitte 66 und 67 des Gehäuses 61 aus dem Werkzeug 60 herausgeführt.

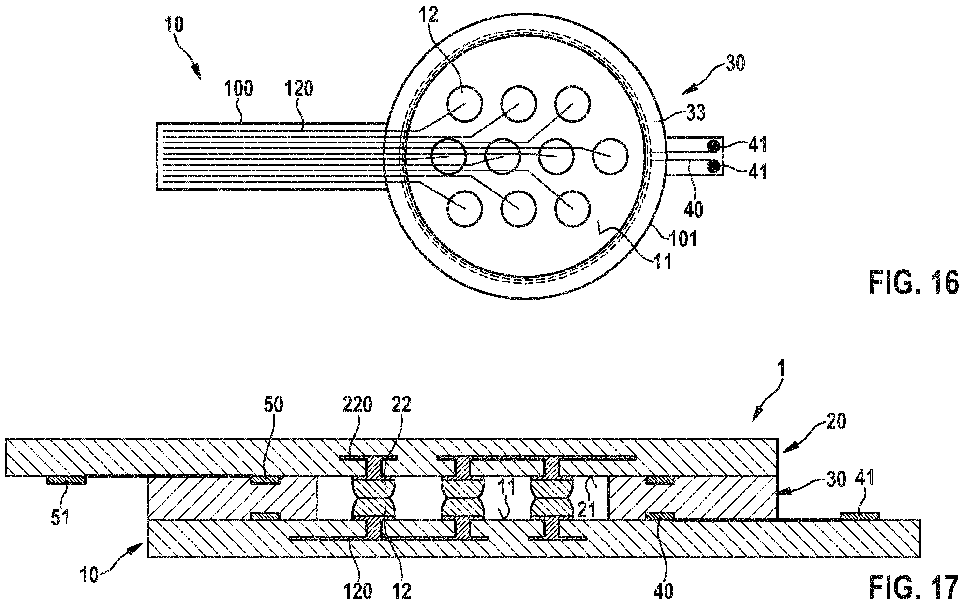

Weiterhin kann die in den Figuren 9 bis 12 gezeigte Ausführungsform weiter vereinfacht werden, indem auf das Dichtungselement 31 verzichtet wird. Die Dichtung 30 wird in diesem Fall gemäß Figuren 16 und 17 durch einen einzelnen, mit den Oberflächen 11, 21 zu verschmelzenden Materialbereich gebildet.

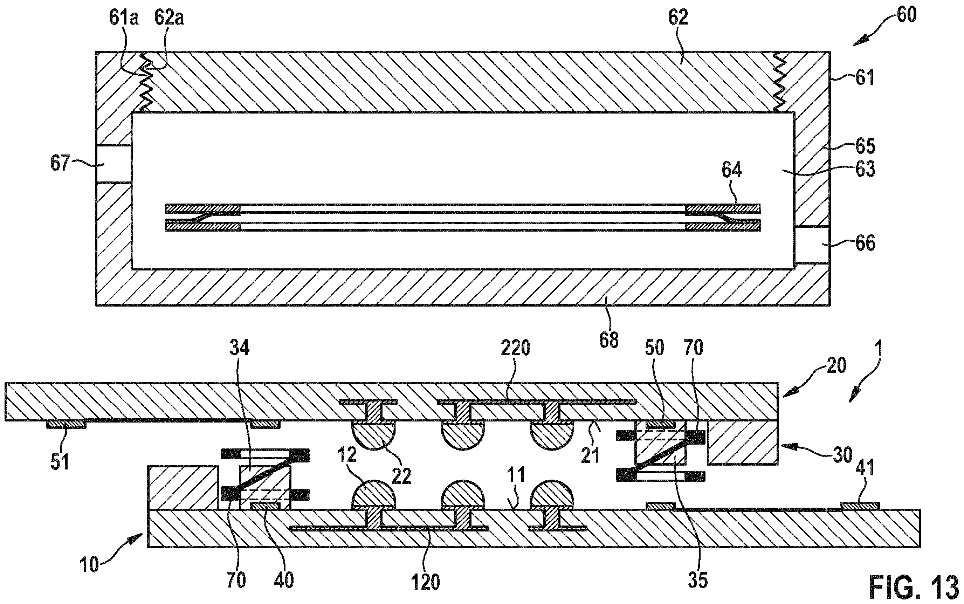

Weiterhin zeigen die Figuren 13 bis 15 eine Abwandlung der in den Figuren 6 bis 12 gezeigten Ausführungsform des Implantats 1, wobei gemäß den Figuren 13 bis 15 anstelle eines elastisch deformierbaren Dichtungselements 31 in Form einer Silikondichtung zwei Federelemente 70 in die Dichtung 30 eingebettet werden.

Hierzu weist das erste Flachkabel 10 einen Materialbereich 34 aus dem thermoplastischen Material auf, der von der Oberfläche 11 absteht und an dem ein Federelement 70 festgelegt ist. Der Materialbereich 34 ist mittels eines unterhalb des Materialbereichs 34 angeordneten Heizleiters 40 aufschmelzbar. In ähnlicher Weise weist das zweite Flachkabel 20 einen von der Oberfläche 21 abstehenden Materialbereich 35 aus dem thermoplastischen Material auf, an dem ebenfalls ein Federelement 70 festgelegt ist. Werden nun die beiden Flachkabel 10, 20 in der zuvor beschriebenen Weise mittels des Werkzeugs 60 gegeneinander gedrückt und dabei die Materialbereiche 34, 35 aufgeschmolzen, vereinigen sich die Materialbereiche 34, 35 mit den Oberflächen 11, 21 und bilden ein umlaufendes Dichtelement 30, in das die Federelemente 70 im vorgespannten Zustand eingebettet werden. Gleichzeitig werden die mechanischen Berührungen der Kontakte 12, 22 realisiert.

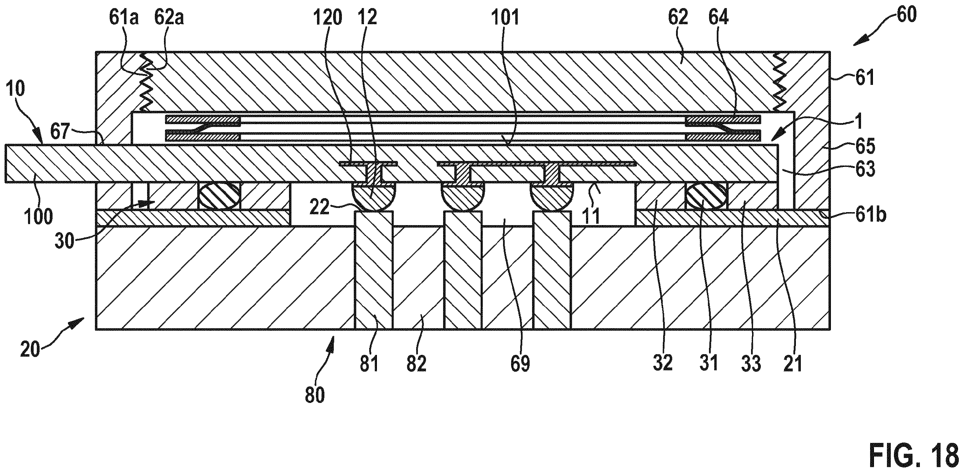

Weiterhin zeigen die Figuren 18 bis 24 Ausführungsformen eines erfindungsgemäßen Implantats, bei denen jeweils die erste Komponente durch ein Flachkabel 10 gebildet ist, während die zweite Komponente 20 ein implantierbares medizinischen Gerät 20 ist (z.B. ein Pulsgenerator), das eine Durchführung 80 aufweist, über die elektrische Leiter 81, die an ihren Enden die Kontakte 22 ausbilden, aus einem Gehäuse des Geräts 20 herausgeführt sind. Hierbei sind die Leiter 81 insbesondere in einen Isolator 82 (vorzugsweise in Form einer Keramik 82) der Durchführung 80 eingebettet, auf dem eine thermoplastische Oberfläche 21 angeordnet ist, mit der wiederum die Dichtung 30 verschmolzen werden kann.

Gemäß Fig. 18 kann die Dichtung 30 vor dem Verbinden mit dem Gerät 20 zwei von der Oberfläche 11 des Flachkabels 10 abragende Materialbereiche 32, 33 aufweisen, die eine Nut definieren, in der ein elastisch deformierbares Dichtungselement 31 integriert ist. Das Flachkabel 10 wird nun mit diesen Materialbereichen 32, 33 gegen eine ebenfalls aus dem thermoplastischen Material der Materialbereiche 32, 33 bestehende Oberfläche 21 des Geräts 20 gedrückt, wobei die Materialbereiche 32, 33 durch Zufuhr extern erzeugter Wärme mit den Oberflächen 11, 21 verschmolzen werden, so dass eine abdichtende Verbindung zwischen dem Flachkabel 10 und dem implantierbaren medizinischen Gerät 20 hergestellt wird. Weiterhin wird hierbei das Dichtungselement 31 gegen die beiden Komponenten 10, 20 vorgespannt. Bei einem späteren Aufschmelzen der Dichtung 30 erleichtert dies das Trennen der beiden Komponenten 10, 20. Beim gegeneinander Drücken der beiden Komponenten 10, 20 des Implantats 1 werden des Weiteren die Kontakte 12 des Flachkabels 10 mechanisch gegen die Kontakte 22 der mittels der Durchführung 80 aus dem Gerät 20 herausgeführten Leiter 81 gedrückt, so dass gleichfalls eine elektrisch leitfähige Verbindung zwischen den Kontakten 12 des Flachkabels 10 und den Kontakten 22 des Geräts 20 hergestellt wird.

Figur 18 zeigt des Weiteren das Werkzeug 60, mit dessen Hilfe der Endabschnitt 101 des Flachkabels 10 mit der Dichtung 30 voran gegen die thermoplastische Oberfläche 21 der Durchführung 80 des Geräts 20 gedrückt wird während die Dichtung 30 bzw. deren Materialbereiche 32, 33 aufgeschmolzen werden. Hierzu weist das Werkzeug 60 wiederum ein Gehäuse 61 mit einer lateralen Wandung 65 auf, wobei jedoch das Gehäuse 61 nunmehr keinen Boden 68 aufweist (vgl. oben), sondern eine Öffnung 69, so dass die Materialbereiche 32, 33 sowie das Dichtungselement 31 der Dichtung 30 zur Anlage an die Oberfläche 21 des Geräts 20 gelangen können. Zum Ausbilden eines Widerlagers für den schraubbaren Kolben 62 wird vorliegend eine Stirnseite 61b des Gehäuses 61 bzw. der Wandung 65, die die besagte Öffnung 69 begrenzt, am Gerät 20 festgelegt, z.B. mittels einer Schweißverbindung. Der besagte Kolben 62 weist, wie oben bereits beschrieben, ein Außengewinde 62a auf, das in ein Innengewinde 61a des Gehäuses 61 des Werkzeugs 60 eingreift. Der Kolben 62 ist damit in eine Schraubbewegung versetzbar, so dass er in Richtung auf den im Innenraum 63 des Werkzeuggehäuses 61 angeordneten Endabschnitt 101 des Flachkabels 10 bewegbar ist. Zwischen dem Kolben 62 und dem Flachkabel 10 ist wie zuvor bevorzugt ein Federelement 64 angeordnet, bei dem es sich z.B. um einen Blattfederring oder eine Membranfeder handeln kann. Der Kolben 62 drückt somit über das Federelement 64 gegen das Flachkabel 10 und drückt dieses gegen das Gerät 20. Hierbei kann die Dichtung 30 wie oben beschrieben aufgeschmolzen werden sowie die elektrische Verbindung zwischen den Kontakten 12, 22 hergestellt werden. Des Weiteren kann die Wandung 65 des Gehäuses einen Ausschnitt 67 aufweisen, über den z.B. der Leitungskörper 100 des Flachkabels 10 aus dem Gehäuse 61 des Werkzeugs 60 herausgeführt werden kann. Nach Herstellung der die beiden Oberflächen 11, 21 verbindenden Dichtung 30 durch Aufschmelzen der Materialbereiche 32, 33 kann das Werkzeug 60 wieder entfernt werden.

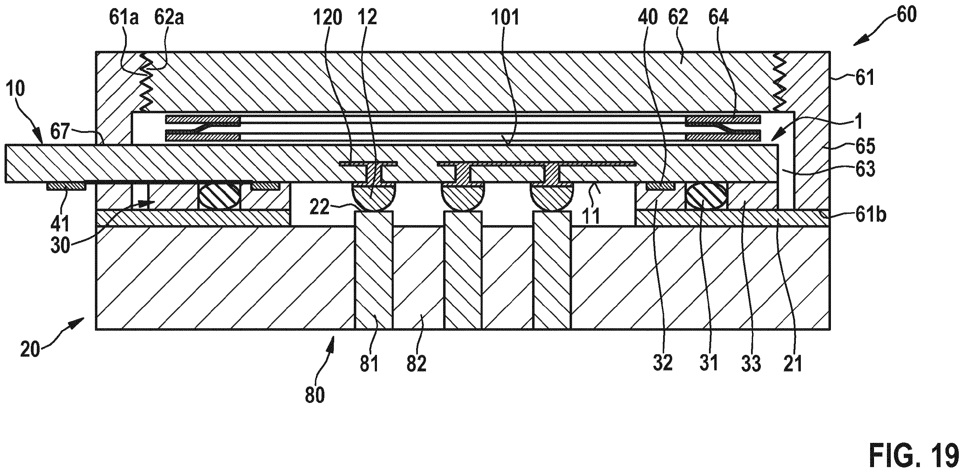

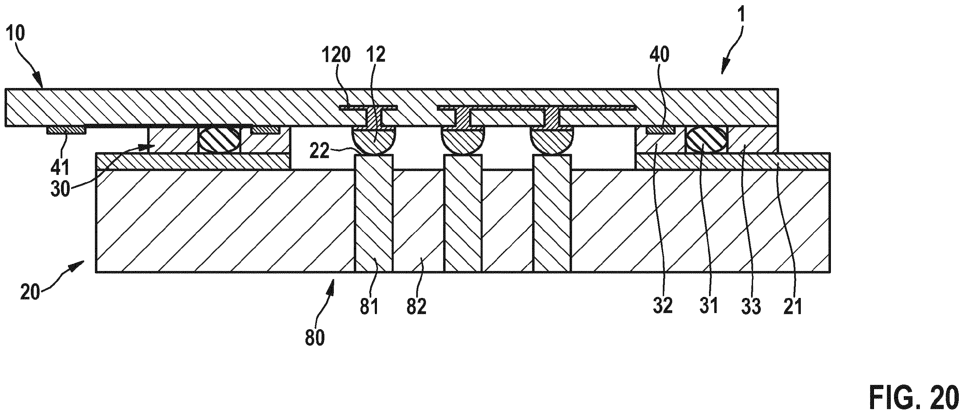

Fig. 19 zeigt eine Abwandlung des in der Figur 18 gezeigten Implantats 1, das aus dem Flachkabel 10 und dem damit verbundenen Gerät 20 gebildet ist. Bei dieser Abwandlung ist zusätzlich ein Heizleiter 40 vorgesehen, der auf der Oberfläche 11 des Flachkabels 10 angeordnet ist und z.B. durch den inneren Materialbereich 32 überdeckt ist. Mittels des Heizleiters 40 kann nun die Dichtung 30 gezielt aufgeschmolzen werden, so dass sich diese mit den beiden Oberflächen 11, 21 verbindet. Kontakte 41 zum Kontaktieren des Heizleiters 40 bzw. zum Anlegen einer Heizspannung an den Heizleiter 40 können z.B. über einen Ausschnitt 67 des Gehäuses 61 bzw. der Wandung 65 des Werkzeugs 60 aus dem Werkzeug 60 herausgeführt sein. Figur 20 zeigt das Implantat 1 mit fertig ausgebildeter Dichtung 30 nach Herausnahme aus dem Werkzeug 60.

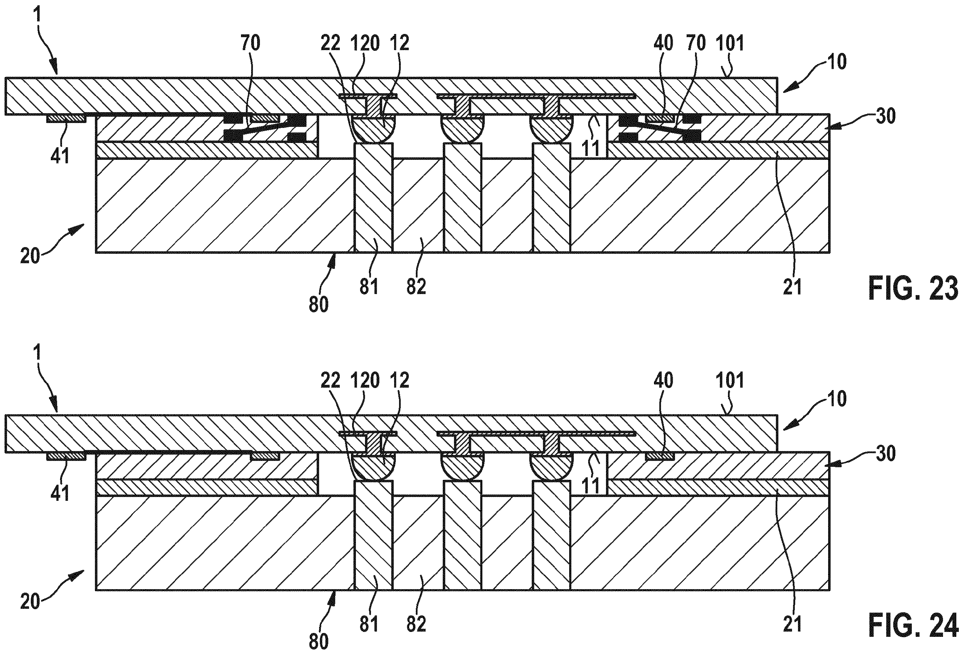

Wie anhand der Fig. 24 ersichtlich ist, kann bei der Ausführungsform gemäß Figur 19 auch gänzlich auf das Dichtungselement 31 verzichtet werden.

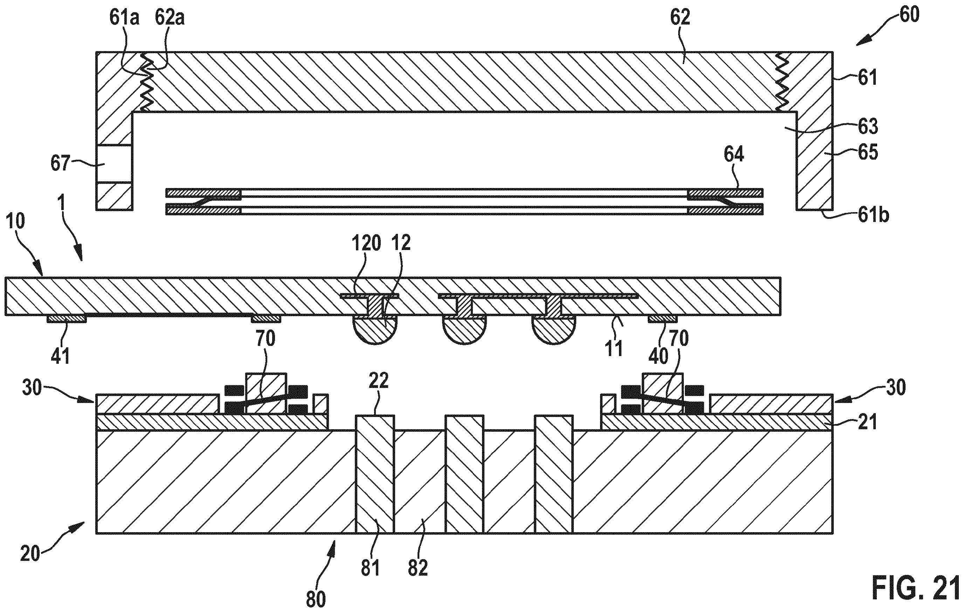

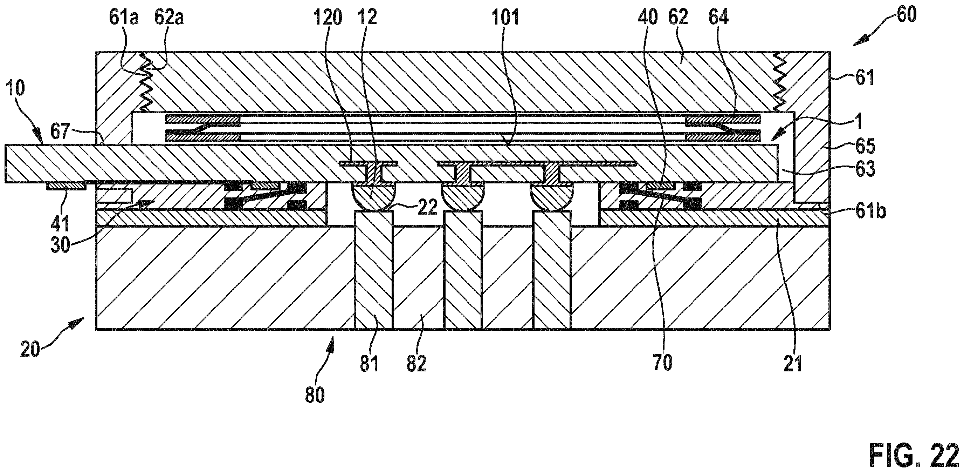

Die Figur 21 zeigt im Zusammenhang mit den Figuren 22 und 23 eine weitere Ausführungsform eines erfindungsgemäßen Implantats, bei der ein Flachkabel 10 mit der Durchführung 80 eines implantierbaren medizinischen Geräts 20 verbunden wird bzw. ist.

Hierbei kann die Dichtung 30 vor dem Verbinden mit dem Flachkabel 10 einen von der thermoplastischen Oberfläche 21 des Geräts 21 abragenden Materialbereich 30 aufweisen, an dem ein oder mehrere Federelemente 70 angeordnet sind. Auf der gegenüberliegenden thermoplastischen Oberfläche 11 des Flachkabels 10 ist hingegen ein Heizleiter 40 angeordnet, der zum Aufschmelzen des Materialbereichs 30 konfiguriert ist, wenn das Flachkabel 10 mittels des Werkzeugs 60 gegen das Gerät 20 gedrückt wird. Das Werkzeug 60 kann dabei so gestaltet sein, wie es oben im Zusammenhang mit der Figur 18 bzw. 19 erläutert worden ist. Figur 22 zeigt den Moment der Herstellung der Dichtung 30, die mit den Oberflächen 11 und 21 abdichtend verschmolzen ist. Hierbei werden auch die Federelemente 70 gegen die Komponenten 10, 20 vorgespannt, was ein späteres Trennen von Flachkabel 10 und Gerät 20 erleichtert, wenn die Dichtung 30 mittels des Heizleiters 40 wieder aufgeschmolzen wird. Figur 23 zeigt das Implantat 1 nach Herausnahme aus dem Werkzeug 60.

Die Erfindung ermöglicht mit Vorteil eine vereinfachte mechanische Konstruktion des Implantats 1, wobei mit einer Dichtung 30 ein gesamtes Elektrodenarray 12, 22 abgedichtet werden kann. Durch die Erfindung wird somit die Konstruktion von lösbaren Steckern/Flachkabeln für Implantate deutlich vereinfacht und die Kosten für die Verbindungstechnik können deutlich reduziert werden. Die Dichtung kann sich in vorteilhafter Weise bereits direkt auf einer Oberfläche einer Komponente befinden. Der Heizleiter kann benachbart dazu angeordnet sein.

## Patentansprüche

1. Medizinisches Implantat (1), mit:
- einer ersten Komponente (10) aufweisend eine Oberfläche (11), auf der eine Mehrzahl an elektrischen Kontakten (12) angeordnet ist,
- einer zweite Komponente (20) aufweisend eine Oberfläche (21) sowie eine Mehrzahl an elektrischen Kontakten (22), wobei jeder Kontakt (12) der ersten Komponente (10) einen zugeordneten Kontakt (22) der zweiten Komponente (20) elektrisch leitend kontaktiert, und
- einer Dichtung (30, 32, 33, 43, 35), die zum Abdichten der Kontakte (12, 22) zwischen den beiden Oberflächen (11, 21) angeordnet ist,
**dadurch gekennzeichnet,**
**dass** die Dichtung (30) und die beiden Oberflächen (11, 21) aus einem thermoplastischen Material gebildet sind, wobei die Dichtung (30, 32, 33, 34, 35) mit den beiden Oberflächen (11, 21) zum Abdichten der Kontakte (12, 22) verschmolzen ist und zum voneinander Trennen der beiden Komponenten (10, 20) aufschmelzbar ist.

2. Medizinisches Implantat nach Anspruch 1, **dadurch gekennzeichnet, dass** das medizinische Implantat (1) zum Trennen der beiden Komponenten (10, 20) zumindest ein Heizelement (40), insbesondere einen Heizleiter (40) aufweist, der dazu ausgebildet ist, das thermoplastische Material der Dichtung (30, 32, 33, 34, 35) aufzuschmelzen.

3. Medizinisches Implantat nach Anspruch 2, **dadurch gekennzeichnet, dass** das mindestens eine Heizelement (40) in die Dichtung (30, 32, 33, 34, 50) eingebettet ist oder auf einer der Oberflächen (11, 21) angeordnet ist.

4. Medizinisches Implantat nach einem der vorhergehenden Ansprüche, **dadurch gekennzeichnet, dass** die Dichtung (30, 32, 33) ein elastisch deformierbares Dichtungselement (31) oder Federelement (70) aufweist, das gegen die beiden Komponenten (10, 20) vorgespannt ist.

5. Medizinisches Implantat nach einem der vorhergehenden Ansprüche, **dadurch gekennzeichnet, dass** die erste Komponente (10) durch ein erstes Flachkabel gebildet ist, und dass die zweite Komponente (20) durch ein zweites Flachkabel gebildet ist.

6. Medizinisches Implantat nach einem der Ansprüche 1 bis 4, **dadurch gekennzeichnet, dass** die erste Komponente (10) durch ein Flachkabel gebildet ist und dass die zweite Komponente (20) durch ein implantierbares medizinisches Gerät gebildet ist, wobei die Kontakte (22) der zweiten Komponente (20) Kontakte (22) einer Durchführung (80) des medizinischen Geräts (20) sind.

7. Verfahren zur Herstellung eines medizinischen Implantats (1), aufweisend die Schritte:
- Bereitstellen einer ersten und einer zweiten Komponente (10, 20) des medizinischen Implantats (1), wobei die erste Komponente (10) eine Oberfläche (11) aufweist, auf der eine Mehrzahl an elektrischen Kontakten (12) angeordnet ist, und wobei die zweite Komponente (20) eine Oberfläche (21) sowie eine Mehrzahl an elektrischen Kontakten (22) aufweist, wobei die Oberflächen (11, 21) aus einem thermoplastischen Material gebildet sind, und
- Gegeneinander Drücken der beiden Komponenten (10, 20) und Aufschmelzen einer aus dem thermoplastischen Material bestehenden und zwischen den beiden Oberflächen (11, 21) angeordneten Dichtung (30, 32, 33, 34, 35), so dass sich die Dichtung (30, 32, 33) mit den beiden Oberflächen (11, 21) verbindet, und so dass jeder Kontakt (12) der ersten Komponente einen zugeordneten Kontakt (22) der zweiten Komponente (20) elektrisch leitend kontaktiert.

8. Verfahren nach Anspruch 7, wobei die Dichtung (30) zwei aus dem thermoplastischen Material gebildete Materialbereiche (32, 33, 34, 35) aufweist.

9. Verfahren nach Anspruch 8, wobei die Materialbereiche (32, 33) vor dem Aufschmelzen der Dichtung (30, 32, 33, 34, 35) an der Oberfläche (11) der ersten Komponente (10) oder an der Oberfläche (21) der zweiten Komponente (20) festgelegt sind.

10. Verfahren nach Anspruch 8 oder 9, wobei zwischen den beiden Materialbereichen (32, 33) ein elastisch deformierbares Dichtungselement (31) angeordnet wird, wobei das Dichtungselement (31) beim gegeneinander Drücken der beiden Komponenten (10, 20) gegen die beiden Komponenten (10, 20) vorgespannt wird.

11. Verfahren nach einem Ansprüche 8 bis 10, wobei der eine Materialbereich (34) vor dem Aufschmelzen der Dichtung (30) an der Oberfläche (11) der ersten Komponente (10) festgelegt ist, und wobei der andere Materialbereich (35) vor dem Aufschmelzen der Dichtung (30, 34, 35) an der Oberfläche (21) der zweiten Komponente (20) festgelegt ist.

12. Verfahren nach einem der Ansprüche 7 bis 9, 11, wobei beim Aufschmelzen der Dichtung (30, 32, 33, 34, 35) zumindest ein Federelement (70) in die Dichtung (30) eingebettet wird und beim gegeneinander Drücken der beiden Komponenten (10, 20) gegen die Komponenten (10, 20) vorgespannt wird.

13. Verfahren nach einem der Ansprüche 7 bis 12, dass die Dichtung (30) mit Hilfe eines Heizelements (40) aufgeschmolzen wird, der auf der ersten Komponente (10) angeordnet ist und/oder dass die Dichtung (30) mit Hilfe eines Heizelements (50) aufgeschmolzen wird, der auf der zweiten Komponente (20) angeordnet ist.

14. Verfahren nach einem der Ansprüche 7 bis 13, wobei die beiden Komponenten (10, 20) mittels eines Werkzeugs (60) gegeneinandergedrückt werden, das zum Ausüben einer Kraft auf die beiden Komponenten (10, 20) einen schraubbaren Kolben (62) aufweist.

15. Verfahren nach einem der Ansprüche 7 bis 14, wobei die erste Komponente (10) durch ein erstes Flachkabel gebildet ist, und wobei die zweite Komponente (20) durch ein zweites Flachkabel gebildet ist; oder wobei die erste Komponente (10) durch ein Flachkabel gebildet ist, und wobei die zweite Komponente (20) durch ein implantierbares medizinisches Gerät (20) gebildet ist, wobei die Kontakte (22) der zweiten Komponente (20) Kontakte (22) einer Durchführung (80) des medizinischen Geräts (20) sind.
